# EUROPEAN PATENT APPLICATION

(11) **EP 4 342 989 A1**
(43) Date of publication of application: **27.03.2024**
(21) Application number: 22804768.4
(22) Date of filing: 20.05.2022
(51) Int. Cl.: C12N 15/13, C07K 16/10, C12M 1/34, C12N 1/15, C12N 1/19, C12N 1/21, C12N 5/10, C12N 15/63, C12P 21/08, C12Q 1/02, G01N 33/569

(54) **ANTI-NOROVIRUS ANTIBODY**

(30) Priority: 20.05.2021 JP 2021084985
(71) Applicant: Denka Company Limited, Tokyo 103-8338 (JP)
(72) Inventor: SAITO, Yuji, Gosen-shi, Niigata 959-1834 (JP); HIRAOKA, Koji, Gosen-shi, Niigata 959-1834 (JP); OGASAWARA, Shinya, Machida-shi, Tokyo 194-8560 (JP)
(74) Representative: Plougmann Vingtoft a/s
(86) International application number: PCT/JP2022/020944
(87) International publication number: WO 2022/244860

(57) **Abstract**

Disclosed is an anti-norovirus antibody that reacts with noroviruses of many genotypes and can collectively detect the noroviruses. The anti-norovirus antibody or an antigen-binding fragment thereof undergoes antigen-antibody reaction with the peptides composed of the amino acid sequences of SEQ ID NO: 1 and SEQ ID NO: 2, respectively. The anti-norovirus antibody, which can broadly bind to noroviruses and detect a wide range of human noroviruses with high specificity and sensitivity, and a norovirus immunoassay and a norovirus immunoassay device, both using the anti-norovirus antibody, are provided.

## Description

### TECHNICAL FIELD

The present invention relates to an anti-norovirus antibody, and an immunoassay of norovirus and an immunoassay device of norovirus both using the same.

### BACKGROUND ART

Human norovirus infection occurs orally and causes infectious gastroenteritis as the norovirus grows in the gastrointestinal tract from the duodenum to the upper part of the small intestine. Norovirus infection causes drop off of small intestinal epithelial cells in the vicinity of the duodenum, resulting in symptoms such as vomiting, diarrhea, and abdominal pain. The incubation period of norovirus from infection to the onset of symptoms is from 12 hours to 72 hours (one to two days on average). Even after the symptoms are gone, fecal shedding of the virus continues for one to three weeks, and even a shedding period of more than seven weeks has been reported. Norovirus infection is responsible for about seven tenths of the patients with food poisoning incidents.

Noroviruses are non-enveloped viruses containing a genome of single-stranded positive-sense RNA of approximately 7500 bases. The norovirus genome is reported to contain three protein-coding regions (ORFs), that is, ORF1 encoding a non-structural protein involved in viral replication, ORF2 encoding a structural capsid protein (VP1), and ORF3 encoding a minor structural protein (VP2). Noroviruses are classified into five groups, genogroups I to V (GI to GV), based on the sequence similarity of capsid genes. Most noroviruses that are infectious to humans belong to GI, GII, and GIV. Among these noroviruses, genogroups I (GI) and II (GII) noroviruses are rich in genetic diversity, and various viruses from different phylogenetic lineages are detected in human samples. It is believed that genogroup I noroviruses can be classified into nine or more genotypes (genetic types) and genogroup II noroviruses can be classified into 22 or more genotypes.

The capsid structural protein is detected with an antibody by enzyme immunoassay (EIA) (see Non-Patent Document 1) or immunochromatography (see Non-Patent Document 2) to detect noroviruses. Therefore, accurate detection of a norovirus antigen requires an antibody that reacts to all norovirus genotypes.

However, an antibody that recognizes and reacts to an antigenic region common to all genotypes is not readily available. Therefore, to date, a reagent comprising a combination of multiple antibodies against a norovirus antigenic peptide having a specific amino acid sequence or to a fragment thereof (see, for example, Patent Document 1) has been prepared, and different genotypes of noroviruses have been individually detected using the reagent.

For example, in a study to obtain an antibody reactive to all GII genogroup noroviruses, it has been reported that an antibody binding to a specific site in the protruding domain of the GII norovirus capsid binds broadly to GII noroviruses and specifically detects noroviruses of nearly all genotypes (GII/1 to GII/17) belonging to GII (see Patent Document 2).

However, norovirus is so mutable, with many variations found in the protruding domain exposed on the capsid surface, and therefore there has been a problem that a once-prepared antibody that undergoes antigen-antibody reaction with protrusions across a wide range of genotypes may lose reactivity to newly generated mutant strains.

Therefore, there was a need to develop an antibody capable of collectively detecting many noroviruses of different genotypes.

On the other hand, Non-Patent Document 3 describes a monoclonal antibody that undergoes antigen-antibody reaction with the shell domain of norovirus capsid.

### PRIOR ART DOCUMENT

### PATENT DOCUMENTS

Patent Document 1: JP 2009-542715 A
Patent Document 2: WO 13/039165

### NON-PATENT DOCUMENTS

Non-Patent Document 1: "Evaluation of an improved norovirus antigen detection EIA kit", Japanese Journal of Medicine and Pharmaceutical Science, Vol. 61, No. 1, Page. 93-98 (2009.01.25)
Non-Patent Document 2: "Evaluation of the QuickNavi-Noro quick norovirus antigen diagnosing agent", Japanese Journal of Medicine and Pharmaceutical Science, Vol. 61, No. 5, Page. 779-785 (2009.05.25)
Non-Patent Document 3: Gabriel I. Parra et al., PLOS ONE, June 2013, Volume 8, Issue 6, e67592

### SUMMARY OF THE INVENTION

### PROBLEMS TO BE SOLVED BY THE INVENTION

The present invention pertains to providing an anti-norovirus antibody that reacts with noroviruses of many genotypes and can collectively detect the noroviruses.

### MEANS FOR SOLVING THE PROBLEMS

In the present invention, a study was conducted to obtain an antibody that reacts commonly with noroviruses in the context of the problem described above, focusing on an internal structure, the shell domain, rather than the protruding domain exposed on the surface of the norovirus capsid, and found that an antibody that binds to a specific site in the shell domain of the norovirus capsid can bind broadly to noroviruses and specifically detect a wide range of human noroviruses.

That is, the present invention provides the following:
(1) An anti-norovirus antibody which undergoes antigen-antibody reaction with the peptides composed of the amino acid sequences of SEQ ID NO: 1 and SEQ ID NO: 2, respectively, or an antigen-binding fragment thereof.
(2) The anti-norovirus antibody of claim 1 or an antigen-binding fragment thereof, which undergoes antigen-antibody reaction with the peptides composed of the amino acid sequences of SEQ ID NO: 3 and SEQ ID NO: 4, respectively.
(3) The anti-norovirus antibody of (1) or (2) or an antigen-binding fragment thereof, wherein the anti-norovirus antibody is a monoclonal antibody.
(4) An immunoassay of norovirus utilizing the antigen-antibody reaction between norovirus in a sample and the anti-norovirus antibody of any of (1) to (3) or an antigen-binding fragment thereof.
(5) An immunoassay device of norovirus, comprising the anti-norovirus antibody of any of (1) to (3) or an antigen-binding fragment thereof.

### EFFECTS OF THE INVENTION

By the present invention, an anti-norovirus antibody which can broadly bind to noroviruses and detect a wide range of human noroviruses with high specificity and sensitivity, as well as an immunoassay of norovirus and an immunoassay device of norovirus, both using the anti-norovirus antibody, was provided.

### MODE FOR CARRYING OUT THE INVENTION

An anti-norovirus antibody according to the present invention is an antibody that undergoes antigen-antibody reaction with the peptides composed of the amino acid sequences of MMMASKDAPPSMDGASGAGQLVP (SEQ ID NO: 1) and MKMASNDAAPSNDGAAGLVPE (SEQ ID NO: 2), respectively, and binds to an epitope in the shell domain of norovirus capsid structural protein. The epitope is considered to be present in the sequences MMMASKDAPPSMDGASG (SEQ ID NO: 3) and MKMASNDAAPSNDGAAG (SEQ ID NO: 4), based on the commonality between the amino acid sequences of SEQ ID NOs: 1 and 2. It should be noted that this region is different from the epitope region of the monoclonal antibody described in Non-Patent Document 3.

The norovirus capsid structural protein (VP1) is known to comprise the shell domain (S domain) and the protruding domain (P domain). The S domain is thought to be responsible for VP1 assembly. On the other hand, the P domain is further divided into the P1 and P2 subdomains. It has been reported that the P1 subdomain interacts with the S domain and increases the physical stability of the capsid, and that the P2 subdomain is located on the outermost surface of the virus particle and is targeted by a neutralizing antibody against mouse norovirus.

The anti-norovirus antibody of the present invention can be of any desired form, such as IgG, IgA, IgY, IgD, IgM, IgE, or one or more portions thereof, such as heavy chain, light chain, Fc, or F(ab) region.

The anti-norovirus antibody used in the present invention can be obtained as a polyclonal or monoclonal antibody using a known technique. A monoclonal antibody of mammalian origin includes monoclonal antibodies produced in hybridoma cells and monoclonal antibodies produced by host cells transformed with a bioengineered expression vector containing a gene for the antibody.

A hybridoma producing the anti-norovirus monoclonal antibody can be produced essentially by using a known technique as described below:
A recombinant norovirus virus-like particle (VLP) is used as a sensitizing antigen for immunization according to a conventional immunization method, and the resulting immune cells are fused with known parental cells by a conventional cell fusion technique;
monoclonal antibody-producing cells are screened by a conventional screening technique to find a hybridoma producing a monoclonal antibody that undergoes antigen-antibody reaction with the peptides composed of the amino acid sequences of SEQ ID NO: 1 and SEQ ID NO: 2, respectively, among other monoclonal antibodies. In addition, a polyclonal antibody can be produced by immunizing an animal such as a mouse, rat, or hamster with at least one of the peptides having the amino acid sequences of SEQ ID NO: 1 and SEQ ID NO: 2, respectively, directly or with at least one of the peptides immobilized on a regularly used carrier, and recovering the polyclonal antibody from the serum of the animal.

The recombinant norovirus VLP can be obtained by integrating the sequences of the norovirus capsid genes into a transfer plasmid vector, transfecting Sf9 cells with baculovirus DNA and the above plasmid together to generate a recombinant virus via homologous recombination, propagating the recombinant virus to prepare a seed virus, using the seed virus for protein expression in Tn5 cells, and then purifying the recombinant VLP of interest from the cells or the culture supernatant by a known technique. It should be noted that recombinant norovirus VLP is known *per se* and is widely used for anti-norovirus antibody development and the like.

A mammal to be immunized with the sensitizing antigen is not limited to a specific animal species, but is preferably selected considering the compatibility with parental cells used for the cell fusion, and rodents, such as mouse, rat, and hamster, are generally used.

The sensitizing antigen is administered to an animal for immunization in accordance with a known method. For example, the sensitizing antigen is injected intraperitoneally or subcutaneously into a mammal. Specifically, the sensitizing antigen is diluted or suspended in an appropriate volume of PBS (phosphate-buffered saline), physiological saline, or the like, and the resulting dilution or suspension is optionally mixed with an appropriate volume of a typical adjuvant, such as Freund's complete adjuvant, and the resulting mixture is emulsified and then administered, for example, subcutaneously, intradermally, or intraperitoneally to provide a primary stimulation. The same process is then repeated as necessary. The amount of the antigen to be administered is appropriately determined according to the route of administration and animal species, and a typical dosage is preferably about 10 µg to 1 mg per dose. After the level of an antibody of interest is found to be elevated in the serum following the immunization described above, a polyclonal antibody can be obtained by collecting the blood from the mammal and purifying the serum component. The serum component can be purified using, for example, an affinity column on which the sensitizing agent is immobilized.

Immune cells are harvested from the mammal with elevated levels of the antibody and used for cell fusion to produce a monoclonal antibody. Specifically, spleen cells are preferred as the immune cells used for cell fusion.

Mammalian myeloma cells of various known cell lines, such as P3X63, NS-1, MPC-11, and SP2/0, can be appropriately used as parental counterpart cells to be fused with the above immune cells.

The above immune cells can be fused with myeloma cells according to a known method, such as Kohler's method (Kohler et al., Nature, vol. 256, p. 495-497 (1975)). That is, fused cells (hybridomas) are produced by mixing the immune cells with myeloma cells in the presence of a cell fusion promoter, such as polyethylene glycol (PEG, average molecular weight: 1000-6000; concentration: 30-60%) or Sendai virus (HVJ) in a nutrient culture medium such as RPMI1640 or MEM culture medium, optionally supplemented with an additive such as dimethyl sulfoxide.

The hybridomas produced by cell fusion are cultured for one to seven days in a selective medium, such as a medium containing hypoxanthine, thymidine, and aminopterin (HAT medium), and separated from unfused cells. The resulting hybridomas are further screened based on the characteristics of the antibodies produced by the hybridomas. Selected hybridoma are cloned according to a known limiting dilution method to establish monoclonal antibody-producing hybridoma clones.

A known technique can be used as the method to determine the activity of the antibody produced by each of the hybridoma clones. Examples of the technique include ELISA, agglutination assay, and radioimmunoassay.

Examples of the methods used for preparing a monoclonal antibody from each obtained clone of hybridoma cells include a method in which hybridoma cells are cultured according to a conventional method to obtain a monoclonal antibody in the culture supernatant, and a method in which hybridoma cells are administered to a compatible mammal and propagated in the mammal to obtain a monoclonal antibody in the ascites.

Antibodies can be purified using a known purification technique, such as salting out, gel filtration, ion exchange chromatography, or affinity chromatography.

Norovirus in a sample can be specifically detected by applying the anti-norovirus antibody of the present invention to an arbitrary immunoassay.

Immunoassay is known *per se,* and any well-known immunoassay may be used as the immunoassay of the present invention, except that the above anti-norovirus antibody of the present invention or an antigen-binding fragment thereof is used as an antibody or an antigen-binding fragment thereof. Accordingly, the immunoassay is not limited to a specific method and any well-known immunoassay, such as sandwich assay, immunoagglutination assay, or competitive assay, can be employed. Among these assays, a sandwich assay using an anti-norovirus antibody and a labeled anti-norovirus antibody is preferred, and a sandwich assay using an immobilized anti-norovirus antibody and a labeled anti-norovirus antibody is even more preferred.

An insoluble support such as a polystyrene plate, latex beads, magnetic beads, glass fiber membrane, nylon membrane, nitrocellulose membrane, or cellulose acetate membrane is preferred as a support for immobilizing the anti-norovirus antibody. Methods for immobilizing an antibody are well known.

A known label, such as a radioisotope (for example, ³²P, ³⁵S, ³H), an enzyme (for example, peroxidase, alkaline phosphatase, luciferase), a protein (for example, avidin), a low molecular weight compound (for example, biotin), a fluorescent substance (for example, FITC), a chemiluminescent substance (for example, acridinium), a latex bead (for example, colored latex bead, fluorescent latex bead), a colloidal metal particle (for example, noble metal such as gold, silver, or platinum), or carbon atom, can be used in the labeled anti-norovirus antibody.

Norovirus in a sample is detected by reacting the norovirus in the sample with an immobilized anti-norovirus antibody. In the case of a sandwich assay, norovirus can be detected by reacting a fluid containing a sample with an immobilized anti-norovirus antibody and then with the labeled antibody, or by reacting a fluid containing a sample simultaneously with the immobilized anti-norovirus antibody and the labeled antibody. After completion of the reaction, the amount of norovirus in the sample can be determined by measuring the amount of label in the complex formed by the norovirus in the sample, the immobilized anti-norovirus antibody, and the labeled antibody. The amount of label can be measured by a means appropriate to the type of the label. For example, in cases where an enzyme or avidin is used as the label, a substrate is added after the reaction is complete to measure the enzymatic activity. In cases where a fluorescent substance (including a fluorescent latex bead) or chemiluminescent substance is used as a label, the signal from the label is measured under conditions that prevent quenching. The signal from a colored latex bead, a colloidal metal particle, a carbon particle, or the like is measured, for example, by visual observation or reflected light.

Among the immunoassays described above, ELISA and immunochromatography assay are more preferred.

An immunoassay device using the anti-norovirus antibody of the present invention comprises the anti-norovirus antibody of the present invention in an immobilized form. The immunoassay device may be in the form of a kit comprising, for example, a sample dilution solution, a labeled anti-norovirus antibody, a reaction substrate, and the like in addition to the immobilized anti-norovirus antibody.

### EXAMPLES

### Example 1 Production of Anti-Norovirus Monoclonal Antibody

BALB/c mice were immunized with norovirus virus-like particles (VLPs) produced by a conventional method and raised for a period of time. The spleen was removed from the mice, and cells were fused with mouse myeloma cells according to Kohler's method (Kohler et al., Nature, vol. 256, p. 495-497 (1975)). The resulting fused cells (hybridomas) were maintained in an incubator at 37°C, and the resulting culture supernatant from each hybridoma culture was used to study the reactivity of a produced antibody against norovirus. VLPs were diluted to 0.1 µg/mL in 140 mM NaCl, 2.7 mM KCl, 10 mM Na₂HPO₄, 1.8 mM KH₂PO₄, pH 7.3 (hereinafter abbreviated as PBS). The diluted VLP in an amount of 100 µL of VLP/PBS solution, pH 7.3, was added to each well of a plastic microtiter plate (Nunc-Immuno Module F8 Maxisorp Surface plate, trade name, manufactured by Nalgen Nunc International) and the VLPs were immobilized on the microtiter plate at 4°C for 12 hours. After the 12 hours, the VLP/PBS solution in each well was removed by decantation, and a solution of PBS with 0.05% (v/v) Tween 20 (hereinafter abbreviated as PBS-T; Tween: trade name) was added to each well of the microtiter plate in an amount of 200 µL/well, and the PBS-T was removed by decantation. This washing process was repeated a total of three times.

Then, a solution of 1% Perfect-Block (trade name, a blocking agent manufactured by Funakoshi Co., Ltd.) in PBS, pH 7.3, was added in an amount of 200 µL/well to block the wells of the VLP-immobilized microtiter plate at 4°C for 12 hours. After the 12 hours, the microtiter plate was stored at 4°C. To study the reactivity of an anti-norovirus monoclonal antibody in each culture supernatant, each culture supernatant was added to the VLP-immobilized microtiter plate in an amount of 100 µL/well and incubated at 37°C for 1 hour. The culture supernatant in each well was then removed by decantation, and PBS-T was added in an amount of 200 µL/well and then removed by decantation to wash each well. This washing process was repeated a total of three times.

Then, a peroxidase-conjugated goat anti-mouse immunoglobulins antibody (manufactured by Agilent Technologies Inc.) (hereinafter referred to as "enzyme-labeled antibody") was added to each well in an amount of 100 µl/well (diluted 1/10,000) and incubated at 37°C for 1 hour. The enzyme-labeled antibody in each well was then removed by decantation, and PBS-T was added in an amount of 200 µL/well and then removed by decantation to wash each well. This washing process was repeated a total of three times. A solution of 3,3',5,5'-T-tetramethylbenzidine (hereinafter abbreviated as TMB) was then added in an amount of 100 µL/well as a peroxidase reaction substrate solution, and the microtiter plate was left in the dark at 25°C for 30 minutes. Then, a solution of 313 mM H₂SO₄ was immediately added in an amount of 100 µL/well to stop the enzymatic reaction.

The absorbance was then measured in each well, and the value obtained by subtracting the absorbance at 630 nm from the absorbance at 450 nm was used as an indicator to evaluate the reactivity (Josephy P.D., Mason R.P. et al. (1982) J. Biol. Chem. 257, 3669-3675). Cells were sorted for purity (cloned) with testing the supernatant for antibody activity.

This resulted in cell lines producing anti-norovirus monoclonal antibodies listed in Table 1.

Cells from each obtained cell line were administered intraperitoneally to pristane-treated BALB/c mice, and the ascites containing antibodies was collected approximately two weeks later from each mouse. IgG was purified from the obtained ascites by affinity chromatography using a Protein A column to yield a purified anti-norovirus shell domain antibody (SMoAb).

### Example 2 Anti-Norovirus Antibody Epitope Identification

For each anti-norovirus shell domain antibody (SMoAb) produced in Example 1, an amino acid sequence common to each genotype group of noroviruses was identified, and peptide synthesis was performed based on the identified amino acid sequences to produce peptides having one of the identified amino acid sequences. Each of the resulting peptides was diluted to 0.3 µg/mL in PBS. Each peptide dilution, that is, norovirus peptide/PBS solution, pH7.3, was added in an amount of 100 µL/well to a well of a plastic microtiter plate (Nunc-Immuno Module F8 Maxisorp Surface plate, trade name, manufactured by Nalgen Nunc International) and the norovirus peptides were immobilized on the microtiter plate at 4°C for 12 hours. After the 12 hours, the norovirus peptide/PBS solution in each well was removed by decantation, and PBS-T was added to each well of the microtiter plate in an amount of 200 µL/well, and the PBS-T was removed by decantation. This washing process was repeated a total of three times.

Then, a solution of 1% Perfect-Block (trade name, a blocking agent manufactured by Funakoshi Co., Ltd.) in PBS, pH 7.3, was added in an amount of 200 µL/well to block the wells of the peptide-immobilized microtiter plate at 4°C for 12 hours. After the 12 hours, the microtiter plate was stored at 4°C. To determine the reactivity of each anti-norovirus monoclonal antibody, a solution of the anti-norovirus monoclonal antibody at 1 µg/mL was added to the norovirus peptide-immobilized microtiter plate in an amount of 100 µL/well and incubated at 37°C for 1 hour. The antibody solution in each well was then removed by decantation, and PBS-T was added in an amount of 200 µL/well and then removed by decantation to wash each well. This washing process was repeated a total of three times.

Then, the enzyme-labeled antibody was added to each well in an amount of 100 µl/well (diluted 1/10,000) and incubated at 37°C for 1 hour. The enzyme-labeled antibody in each well was then removed by decantation, and PBS-T was added in an amount of 200 µL/well and then removed by decantation to wash each well. This washing process was repeated a total of three times. A TMB solution was then added in an amount of 100 µL/well as a peroxidase reaction substrate solution, and the microtiter plate was left in the dark at 25°C for 30 minutes. Then, a solution of 313 mM H₂SO₄ was immediately added in an amount of 100 µL/well to stop the enzymatic reaction.

The absorbance was then measured in each well, and the value obtained by subtracting the absorbance at 630 nm from the absorbance at 450 nm was used as an indicator to evaluate the reactivity (Josephy P.D., Mason R.P. et al. (1982) J. Biol. Chem. 257, 3669-3675). The peptides on the microtiter plate were reacted with two SMoAb clones obtained in Example 1 or an anti-norovirus polyclonal antibody (PoAb), and the result was checked using the enzyme-labeled antibody and the peroxidase reaction substrate solution. Table 1 shows the amino acid sequences of the peptides confirmed in the reactions.

**[Table 1]**

| Immobilized peptide | | SMoAb 1 | SMoAb 2 | PoAb |
|---|---|---|---|---|
| Genogroup | Sequence | | | |
| GI | MMMASKDAPPSMDGASGAGQLVP (SEQ ID NO: 1) | 2.771 | 1.7613 | 0.017 |
| GII | MKMASNDAAPSNDGAAGLVPE (SEQ ID NO: 2) | 4.489 | 2.698 | 0.059 |

The SMoAb antibodies of the invention are understood to recognize the sequences MMMASKDAPPSMDGASG (SEQ ID NO: 3) and MKMASNDAAPSNDGAAG (SEQ ID NO: 4), each of which contains many sequences common to Nos. 1 and 8.

### Example 3 Reactivity of Anti-Norovirus Monoclonal Antibody in Immunochromatography (IC)

### 1. Immobilization of anti-norovirus antibody on nitrocellulose membrane

Solutions of a monoclonal antibody reactive with the protruding domain of the GI norovirus capsid (GIPMoAb) and a monoclonal antibody reactive with the protruding domain of the GII norovirus capsid (GIIPMoAb) diluted in purified water at 1.0 mg/mL, and an anti-mouse IgG antibody were prepared. Each antibody was applied at a dispensing rate of 1 µL/cm to draw a test line on a nitrocellulose membrane supported by a PET film. An anti-mouse globulin antibody was similarly applied to draw a control line. The resulting membrane is referred to in this example as an antibody-immobilized membrane.

### 2. Immobilization of anti-norovirus antibody on colored polystyrene particle

Each anti-norovirus shell domain antibody (SMoAb) produced in Example 1 was diluted in purified water to 0.5 mg/ml, and polystyrene particles were added to the dilution at a concentration of 0.1% and stirred. Carbodiimide was added to the mixture to a concentration of 1%, and the resulting mixture was further stirred. After centrifugation, the supernatant was removed and the pellet was resuspended in a solution of 50 mM Tris (pH 9.0) and 3.0% BSA to obtain anti-norovirus antibody-bound colored polystyrene particles. The resulting polystyrene particle is referred to in this example as an antibody-immobilized particle.

### 3. Preparation of test strip

The antibody-immobilized membrane and other components (a backing sheet, an absorbent strip, a sample pad) were laminated together and the resulting laminate was cut into 5 mm wide strips to obtain norovirus test strips. These strips are referred to as test strips in this example.

### 4. Immunoassay

A sample suspension containing arbitrarily diluted norovirus VLPs and antibody-immobilized particles, was applied to each strip and allowed to stand for 15 minutes.

A result was considered positive (+) if color development was visually observed at both sites where the anti-mouse IgG antibody and the anti-norovirus antibody were applied. A result was considered negative (-) if color development was visually observed only at the site where the anti-mouse IgG antibody was applied, but not at the site where the anti-norovirus antibody was applied. In addition, a result was considered invalid if no color development was visually observed at the site where the anti-mouse IgG antibody was applied. The intensity of the color on each line is indicated by the increasing number of plus signs as follows: +++ > ++ > +.

Test results were compared between a test strip of the present invention and a conventional product which was a test strip using a monoclonal antibody against the protruding domain of the norovirus capsid.

**[Table 2]**

| Norovirus | | The present invention | Conventional product |
|---|---|---|---|
| Genotype | GI.1 | + | + |
| | GI.3 | ++ | + |
| | GI.5 | ++ | + |
| | GI.6 | + | - |
| | GI.7 | ++ | - |
| | GI.8 | ++ | - |
| | GI.9 | ++ | + |

Table 2 indicates that the test strip of the present invention reacted to all tested GI genotypes when compared to the conventional product. Additionally, the test strip showed stronger responses than the conventional product, indicating that the antibody has a high binding affinity for the norovirus antigen and confirming that the test strip can detect norovirus with higher sensitivity.

## Claims

1. An anti-norovirus antibody which undergoes antigen-antibody reaction with the peptides composed of the amino acid sequences of SEQ ID NO: 1 and SEQ ID NO: 2, respectively, or an antigen-binding fragment thereof.

2. The anti-norovirus antibody of claim 1 or an antigen-binding fragment thereof, which undergoes antigen-antibody reaction with the peptides composed of the amino acid sequences of SEQ ID NO: 3 and SEQ ID NO: 4, respectively.

3. The anti-norovirus antibody of claim 1 or 2 or an antigen-binding fragment thereof, wherein the anti-norovirus antibody is a monoclonal antibody.

4. An immunoassay of norovirus utilizing the antigen-antibody reaction between norovirus in a sample and the anti-norovirus antibody of claim 1 or 2 or an antigen-binding fragment thereof.

5. An immunoassay device of norovirus, comprising the anti-norovirus antibody of claim 1 or 2 or an antigen-binding fragment thereof.
